# EUROPEAN PATENT APPLICATION

(11) **EP 2 982 742 A1**
(43) Date of publication of application: **10.02.2016**
(21) Application number: 14780039.5
(22) Date of filing: 04.04.2014
(51) Int. Cl.: C12M 1/16, C12M 3/00, C12N 1/12

(54) **PHOTOBIOREACTOR FOR MASS CULTURING OF PHOTOSYNTHETIC MICROORGANISM**

(30) Priority: 05.04.2013 KR 20130037421
(71) Applicant: Inha-Industry Partnership Institute, Incheon 402-751 (KR)
(72) Inventor: LEE, Choul Gyun, Seoul 137-930 (KR); KIM, Z Hun, Incheon 404-813 (KR); LIM, Sang Min, Incheon 403-762 (KR); SEONG, Dong Ho, Seoul 150-106 (KR); HOH, Dong Hee, Incheon 403-784 (KR)
(74) Representative: de Bresser, Sara Jean
(86) International application number: PCT/KR2014/002919
(87) International publication number: WO 2014/163426

(57) **Abstract**

The present invention provides a photobioreactor for the economical and effective mass production of a photosynthetic microorganism, comprising a culture container capable of shape maintenance, which can hold a photosynthetic microorganism to be cultured and a liquid for dispersing the photosynthetic microorganism, wherein the whole or at least a part of the culture container comprises a barrier through which water and nutrients can pass but which limits the diffusion of the photosynthetic microorganism.

## Description

### TECHNICAL FIELD

The present invention relates to photobioreactor for culturing a photosynthetic microorganism, and more particularly to a photobioreactor using a barrier for mass culture of a photosynthetic microorganism, wherein the barrier allows water, gas, and nutrients to freely pass through, but restricts free diffusion of the photosynthetic microorganism.

### BACKGROUND ART

Photosynthetic unicellular microorganisms may produce various organic substances such as proteins, carbohydrates, and lipids through photosynthesis. In particular, photosynthetic unicellular microorganisms are recently considered as an optimal organism for the purpose of elimination of carbon dioxide, which is a main cause of global warming, as well as production of added value products such as functional polysaccharides, carotenoids, vitamins, and unsaturated fatty acids. In addition, the photosynthetic unicellular microorganisms have been noted in biological energy production to replace fossil fuel, i.e. a limited energy source, because microalgae can fix carbon dioxide and accumulate it into a body as a lipid. Numerous studies have been conducted to produce bioenergy such as biodiesel by using lipids thus accumulated.

However, to practicalize useful resultants obtained by employing microalgae, e.g. removal of carbon dioxide or production of bioenergy, high concentration culture, mass culture, or high concentration mass culture of photosynthetic microorganisms is required. Thus, a technique associated with establishment of a large-scale culture facility is essentially required.

Typically, various types of photobioreactor placed indoors or on lands have been used as a culture facility for culturing photosynthetic microorganisms, however manufacture, maintenance, and operation of the photosynthetic microorganism culturing facility (such as a lighting unit, and a supplement and mixing unit of medium and gas) are costly, so that it is difficult to establish a large-scale facility required for commercialization. Consequently, profitability secure is a most important and prior task for mass culture of photosynthetic microorganisms in a commercialized scale, and it is urgently required to develop a culturing technique which allows high concentration culture with low cost to be performed and facilitates scale-up. Thus, there is an attempt to culture photosynthetic microorganisms on or in water such as sea or lake by using a semipermeable membrane. Such techniques include Japanese Laid-open Patent Publication No. 2007-330215 and Korean Registered Patent No. 991373, etc.

### DISCLOSURE OF THE INVENTION

### TECHNICAL PROBLEM

However, a photobioreactor using the semipermeable membrane as above has a problem in economical efficiency because the cost of the semipermeable membrane is high; durability is low; and exchange of materials through the semipermeable membrane is restrictive.

The present invention is to solve various problems including the problem as above, and is intended to provide a photobioreactor for mass culture of photosynthetic microorganisms that is efficient in terms of costs, time, efficiency, and space. However, these solutions are for illustrative purpose only, and the scope of the present invention is not limited thereto.

### TECHNICAL SOLUTION

According to one aspect of the present invention, provided is a photobioreactor including a culture container capable of holding a photosynthetic microorganism to be cultured and liquid for dispersing the photosynthetic microorganisms, wherein a whole or at least a part of the culture container has a barrier allowing water, gas, and nutrients to freely pass through, but restricting free diffusion of the photosynthetic microorganisms.

The photosynthetic microorganisms may be dispersed and cultured in the liquid.

The barrier may be prepared with a material which allows water, gas, and nutrients to freely pass through, but restricts diffusion of photosynthetic microorganisms. For example, the barrier may be a mesh sheet or perforated sheet.

The mesh sheet may have a fabric structure. Since the mesh sheet having a fabric structure does not restrict penetration of gas, water, and nutritional salts, but free diffusion of photosynthetic microorganisms, the mesh sheet may be applied to mass culture of photosynthetic microorganisms in an economical and convenient way.

In the photobioreactor, an opening size of the mesh sheet may be adjusted depending on a size of a photosynthetic microorganism to be cultured. For example, the opening size may be 0.1 µm to 200 µm, 0.1 µm to 100 µm, 0.2 µm to 50 µm, 0.5 µm to 25 µm, 0.5 µm to 22 µm, 1 µm to 20 µm, 3 µm to 18 µm, or 4 µm to 16 µm. Optionally, the opening size may be 50% to 300%, 70 to 250%, 85% to 200%, 90% to 160%, 100% to 150%, 100% to 140%, 100% to 130% or 110% to 120% of a size of a photosynthetic microorganism to be cultured.

In the photobioreactor, the mesh sheet may be woven with a polymer fiber. The polymer may be a biodegradable polymer or hardly degradable polymer. The biodegradable polymer may be one or at least two selected from the group consisting of polycaprolactone, poly lactic acid, poly(lactic co-glycolic acid) copolymer, cellulose, methyl cellulose, ethyl cellulose, cellulose acetate, curdlan, polyglutamic acid, polylysine, polyhydroxy alkanoate, polyethylene glycol, polyglycolic acid, and polyester, but not limited thereto.

In addition, the hardly degradable polymer may be one or at least two selected from the group consisting of teflon (polytetrafluoroethylene), polyolefine, polyamides, polyacrylate, silicon, poly methyl methacrylate, polystyrene, ethylene vinyl acetate copolymer, polyethylene-maleic anhydride copolymer, polyamide, poly(vinyl chloride), poly(vinyl fluoride), poly(vinyl imidazole), chlorosulphonate polyolefin, polyethylene terephthalate (PET), nylon, low density polyethylene (LDPE), high density polyethylene (HDPE), acryl, polyetherketone, polyimide, polycarbonate, polyurethane, and polyethylene oxide, but not limited thereto.

The mesh sheet differs from a semipermeable membrane in that the semipermeable film restricts penetration of a macromolecule having at least a certain size such as proteins, while the mesh sheet allows a macromolecule, except a material having a cell size, to freely penetrate. The present inventors demonstrate that the mesh sheet can be usefully applied to mass culture of photosynthetic microorganisms by finding an astonishing fact that, even in the case of culturing a photosynthetic microorganism having a size smaller than the opening size of the mesh sheet, the photosynthetic microorganism is not diffused to outside of the culture container, while nutrients including nutrition salts, water, and gas, which are required for culture, freely pass through.

In the photobioreactor, expect the mesh sheet, remaining parts of the culture container may be prepared with a semipermeable or non-permeable and transparent or translucent material.

The perforated sheet differs from a typical semipermeable membrane in that the perforated sheet is prepared by artificially perforating a non-permeable or semipermeable polymer membrane. The perforated sheet may be prepared by irregularly or regularly perforating a polymer membrane by using a micro perforating device. A size of the hole may be adjusted depending on a size of a photosynthetic microorganism to be cultured. For example, a size of the hole may be 0.1 µm to 200 µm, 0.1 µm to 100 µm, 0.2 µm to 50 µm, 0.5 µm to 25 µm, 0.7 µm to 22 µm, 1 µm to 20 µm, 3 µm to 18 µm, or 4 µm to 16 µm. Optionally, a size of the hole may be 50% to 300%, 70 to 250%, 85% to 200%, 90% to 160%, 100% to 150%, 100% to 140%, 100% to 130% or 110% to 120% of a size of a photosynthetic microorganism to be cultured.

The non-permeable polymer membrane may be one or at least two selected from the group consisting of teflon (polytetrafluoroethylene), polyolefine, polyamides, polyacrylate, silicon, poly methyl methacrylate, polystyrene, ethylene vinyl acetate copolymer, polyethylene-maleic anhydride copolymer, polyamide, poly(vinyl chloride), poly(vinyl fluoride), poly vinyl imidazole, chlorosulphonate polyolefin, polyethylene terephthalate (PET), nylon, low density polyethylene (LDPE), high density polyethylene (HDPE), acryl, polyetherketone, polyimide, polycarbonate, polyurethane, and polyethylene oxide, but not limited thereto.

The semipermeable polymer membrane may be prepared with a composite material of a hydrophilic polymer and the polymer fiber which is used to form the non-permeable membrane as above, wherein the hydrophilic polymer may be one or at least two selected from the group consisting of cellulose, methyl cellulose, ethyl cellulose, cellulose acetate, poly vinyl alcohol, cellophane, nitrocellulose and polyester.

In the photobioreactor, the culture container may be provided with one or more inlet, wherein the inlet may be provided with a switching unit which may be in a form of a zipper, a zipper bag, a valve, a check valve, a tub cap, an adhesive tape, a clip, or a clawclip.

In the photobioreactor, the culture container may be allowed to float on a water surface by a buoyant unit or to submerge under the water surface to a certain depth by a sedimentation unit. The buoyant unit may be a floater such as a buoy separately placed outside of the culture container or may be in a shape of an air injection tube which is not separately placed but extended from the culture container. In addition, the sedimentation unit may be a plumb bob coupled to a lower part of the culture container, or an underwater structure placed underwater or under a water surface to allow the culture container to submerge under the water surface in a certain depth.

In that case, the culture container may be an enclosed-type culture container or an open-type culture container having an opened upper face, wherein the open-type culture container may have a raceway-shaped pond structure, and may further include a culture medium circulator to circulate the culture medium. According to an embodiment of the present invention, the open-type culture container may include an upper frame, and a barrier which is coupled to the upper frame to hold photosynthetic microorganisms. A whole or part of the barrier is prepared with a material which allows water, gas, and nutrients to freely pass through, but restricts free diffusion of the photosynthetic microorganism. Further, the open-type culture container may additionally include a vertical frame and a lower frame. The upper frame may be prepared with a buoyant material or additionally include a buoyant unit. When the upper frame is prepared with a buoyant material, the frame may be a plastic frame or tube having a vacuum inside or including air or gas capable of providing buoyancy. As necessary, the frame may house the barrier to thereby modulate a depth of the culture container.

Additionally, one end of the culture container may be coupled to the buoyant unit and another end may be coupled to the sedimentation unit.

In the photobioreactor, one face of the culture container may be configured to modulate light energy supplied to photosynthetic microorganisms through a light blocking area. The light blocking area has a light filtering function, so that a certain region of wavelengths, among solar light supplied to the photobioreactor, may selectively penetrate or be blocked. The wavelength region may be, for example, where divided as blue, red or green series, etc., among solar light wavelength. The wavelength region to penetrate or to be blocked may be appropriately selected depending on types of photosynthetic microorganisms to be cultured. A membrane having the light filtering function may be prepared by mixing a plastic or polymer material with a chemical component capable of absorbing a light wavelength at a certain wavelength region. The chemical component may be included in a pigment dye.

In the photobioreactor, the culture container may be configured to be rotated in an axial direction by a force from water or wind through a fan attached to one face of the culture container. The fan may be configured to include two or more fans extending to different directions from each other, and the fans may cross each other. In addition, the fan may have a curve to allow the culture container to be rotated on a vertical axis.

According to another aspect of the present invention, provided is a culture facility for photosynthetic microorganisms including a floating structure including the photobioreactor.

The floating structure includes a partition, which is coupled between the frame and frame and installed to prevent loss of the photobioreactor, wherein the partition separates an inside and outside of the culture facility. The partition may be prepared with various materials such as plastics, wood, plywood, nets, but preferably nets in consideration of costs and free communication of environmental water. The culture facility for photosynthetic microorganisms thus formed has a structure similar to a sort of floating fish cages. A buoyant unit may be attached to the frame of the floating structure formed on a water surface to float the floating structure onto the water surface. The buoyant unit may adjust buoyancy taken into account conditions such as solar light energy and nutritional salts required to culture of photosynthetic microorganisms to be cultured. The buoyant unit may be prepared with various materials such as styrofoam or a plastic vessel which has a vacuum inside or includes air or gas capable of providing buoyancy. Also, the buoyant unit may overlay the upper frame, or the upper frame may be coupled to a separate buoyant unit. When the buoyant unit overlays the upper frame, the buoyant unit may serve as a working space used by an operator to conduct a work in the culture facility. In addition, the floating structure may be configured to include an operator supporting unit where an operator may conduct a management work. The operator supporting unit plays a role as a support on which an operator may conduct a work, and the operator supporting unit may be coupled to the buoyant unit or the underwater or floating facility separated from the buoyant unit. In that case, the photobioreactor may be provided with or without a buoyant unit. For the photobioreactor without a buoyant unit, in order not to allow the photobioreactor to submerge under a water surface too deep, a depth of a bottom face of the floating structure may be appropriately modulated to respond to changes in light intensity or cell concentration.

According to another aspect of the present invention, provided is a method for culturing photosynthetic microorganisms, including preparing the photobioreactor; and introducing, into one or more of the photobioreactor, photosynthetic microorganisms and a culture medium, and then putting the photobioreactor to the culture facility to perform photosynthesis.

In the culturing method, the photosynthetic microorganisms are dispersed and cultured in a culture medium without a carrier.

### ADVANTAGEOUS EFFECTS

According to an embodiment of the present invention as above, it is possible to implement the photobioreactor capable of mass culture of photosynthetic microorganisms in an economical and efficient way. Surely, the scope of the present invention is not limited to the effect.

### BRIEF DESCRIPTION OF THE DRAWINGS

FIG. 1 is an illustration schematically showing a photobioreactor according to a first embodiment of the present invention.
FIGS. 2 and 3 are illustrations schematically showing a photobioreactor according to a second embodiment of the present invention.
FIGS. 4 to 8 are illustrations schematically showing a photobioreactor according to a third embodiment of the present invention.
FIG. 9 is an illustration schematically showing a photobioreactor according to a fourth embodiment of the present invention.
FIG. 10 is an illustration schematically showing a photobioreactor according to a fifth embodiment of the present invention.
FIGS. 11 and 12 are illustrations schematically showing a photobioreactor according to a sixth embodiment of the present invention.
FIG. 13 is an illustration schematically showing a photobioreactor according to a seventh embodiment of the present invention.

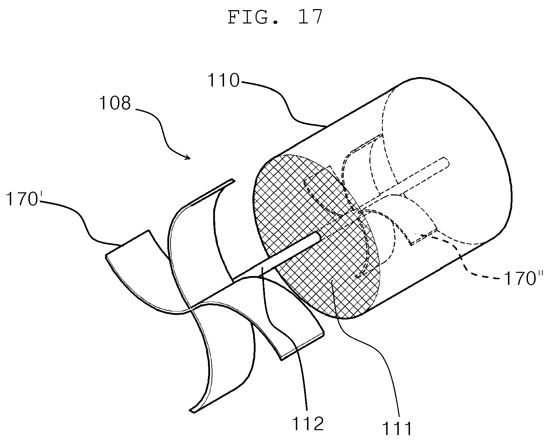

FIGS. 14 to 17 are illustrations schematically showing a photobioreactor according to an eighth embodiment of the present invention.
FIGS. 18 and 19 are illustrations schematically showing a photobioreactor according to a ninth embodiment of the present invention.
FIGS 20 and 21 are illustrations schematically showing a culture facility for photosynthetic microorganisms according to an embodiment of the present invention.
FIG. 22 is an image showing a structure of a mesh sheet according to an embodiment of the present invention taken with a scanning electron microscopy.
FIG. 23 an image showing a process of an experiment to investigate whether photosynthetic microorganisms are released from the photobioreactor according to an embodiment of the present invention.
FIG. 24 is a graph showing a result of analyzing particles sizes and numbers of cells in the culture container to investigate whether photosynthetic microorganisms are released to outside of the culture container of the photobioreactor according to an embodiment of the present invention.
FIG. 25 is a graph showing a result of analyzing particle sizes and number of cells in an outer water bath holding the culture container, after cells are cultured for a certain period of time, to investigate whether photosynthetic microorganisms are released to outside of the culture container of the photobioreactor according to an embodiment of the present invention.
FIG. 26 is an image showing a process of an experiment to compare degrees of growth of photosynthetic microorganisms depending on types of a mesh sheet and semipermeable membrane used in preparation of the photobioreactor according to an embodiment of the present invention.
FIG. 27 is a graph comparing degrees of growth of photosynthetic microorganisms depending on types of a mesh sheet and semipermeable membrane used in preparation of the photobioreactor according to an embodiment of the present invention:
   ●, Δ: polyester mesh sheet;
   ○, ■: nylon mesh sheet;
   ▼, □: 50 kDa molecular weight cut-off semipermeable membrane;
   f/2: f/2 culture medium; and
   NSW: near seawater.
FIG. 28 is a graph showing nutritional salt permeability of the photobioreactor according to an embodiment of the present invention.
FIG. 29 is an illustration schematically showing the photobioreactor according to an embodiment of the present invention used to practically culture algae in sea.
FIG. 30 is an image showing a non-permeable plastic container used to practically culture algae in sea by using the photobioreactor according to an embodiment of the present invention.
FIG. 31 is a schematic diagram schematically showing an experimental design to practically culture algae in sea by using the photobioreactor according to an embodiment of the present invention.
FIG. 32 is a graph showing changes in temperature of seawater and atmosphere when algae are practically cultured in sea by using the photobioreactor according to an embodiment of the present invention.
FIG. 33 is a graph showing daily photosynthetically active radiation (PAR) when algae are practically cultured in sea by using the photobioreactor according to an embodiment of the present invention, and FIG. 34 is a graph showing light irradiation time when algae are practically cultured in sea by using the photobioreactor according to an embodiment of the present invention.
FIG. 35 is a graph showing cell concentration obtained when photosynthetic microorganisms are practically cultured in sea by using the photobioreactor according to an embodiment of the present invention, and FIG. 36 is a graph showing fresh cell wet weight obtained when photosynthetic microorganisms are practically cultured in sea by using the photobioreactor according to an embodiment of the present invention.
FIG. 37 is a graph comparing nitrate permeability of a reused mesh sheet, which is used in the photobioreactor according to an embodiment of the present invention, with that of the photobioreactor prepared by using a semipermeable membrane.

### BEST MODE FOR CARRYING OUT THE INVENTION

The terms used herein are defined as follows.

As used herein, the term "semipermeable" refers to a phenomenon in which only some materials are available to selectively pass through an interface such as a membrane or plate, and counteracts "permeable" indicating that most of materials are available to pass through and "non-permeable" indicating that most of materials are unavailable to pass through.

As used herein, the term "translucent" refers to a phenomenon in which some of light pass through an interface such as a membrane or plate, and counteracts "transparent" indicating that most of light pass through, and "opaque" indicating that pass of light is substantially blocked.

As used herein, the term "barrier" refers to a structure spatially separating an inside of a culture container including photosynthetic microorganisms to be cultured from an outside of the culture container. The wording "allows water, gas, and nutrients to freely pass through, but restricts diffusion of photosynthetic microorganisms" means that most of materials including macromolecules such as water, gas and nutrients are available to freely pass through, rather than a certain molecule selectively passes through by osmosis, however free diffusion of cells such as photosynthetic microorganisms is restricted. Although some cells may pass through the barrier, cell concentrations of both sides of the barrier do not reach equivalent states. A semipermeable membrane differs from "the barrier allowing water, gas, and nutrients to freely pass through, but restricting diffusion of photosynthetic microorganisms" in that the semipermeable membrane restricts penetration of gas, and considerable number of macromolecules is not available to pass through at all. The barrier may be, for example a mesh sheet or perforated sheet.

As used herein, the mesh sheet may be woven with a pattern, for example plain weave, twill weave, and warp stain to include a structure woven by crossing weft threads and warp threads in a vertical direction, or be prepared by varying processing methods or types of a sheet material used such as compound weaving, pile weaving, and leno weaving. The mesh sheet refers to a sheet prepared by applying a technique used in preparation of a woven fabric.

As used herein, the term "perforated sheet" refers to a sheet having holes by artificially perforating a planar material, wherein the planar material may be a film and the film may be a non-permeable or semipermeable membrane. Through artificial perforation, the perforated sheet may provide the same effect as the mesh sheet.

As used herein, the term "nutrient" is a material taken by an organism for nutrition, and includes mineral salts such as ferric salts, sodium salts, potassium salts, phosphate, and magnesium salts, and organic nutrients such as vitamins, proteins, lipids, and carbohydrates except oxygen for respiration and water and carbon dioxide for photosynthesis.

As used herein, the term "environmental water" refers to water in a space where the photobioreactor of the present invention is introduced and culture is performed, wherein the water may include seawater, fresh water and brackish water, as well as water in an artificially created water storage tank or pond.

As used herein, the term "free pass" or "free diffusion" refers to a state in which a certain material is available to pass through two spaces separated by a barrier without limitation, wherein pass is a concept irrelevant to a concentration difference of a certain material in both spaces, while diffusion refers to a phenomenon in which a certain material migrates from a space having a high concentration into a space having a low concentration.

As used herein, the wording "restricting free diffusion" is a case where diffusion of a certain material, which is present in overwhelmingly high concentration in one space among two spaces separated by a barrier, into the other space is substantially restricted under the barrier exiting condition, although diffusion occurs when the barrier does not exist, and the case does not exclude migration of some materials. Thus, the wording "restricting free diffusion" may be used as the same concept of the wording "substantially restricting diffusion".

As used herein, the term "opening size" refers to a size of a space between weft threads and warp threads which are woven to cross each other in the mesh structure.

As used herein, the term "photosynthetic microorganism" refers to green algae, red algae, and blue-green algae, which are capable of photosynthesis, for example chlorella, *Chlamydomonas, Haematococcus, Botryococcus, Scenedesmus, Spirulina, Tetraselmis,* and *Dunaliella,* etc., but not limited thereto. The photosynthetic microorganism as described above may produce metabolites such as carotenoids, mycobiont, phycobiliproteins, lipids, carbohydrates, unsaturated fatty acids, and proteins in a culture container.

Hereinafter, embodiments of the present invention are described with reference to the accompanying drawings. The present invention may, however, be embodied in various forms differs from each other, and should not be construed as limited to the embodiments illustrated in the disclosed drawings. Rather, the embodiments illustrated in drawings are provided so that the disclosure of the present invention will be complete, and will fully convey the scope of the present invention to those skilled in the art. Also, the dimensions of elements may be exaggerated or reduced for convenience of illustration.

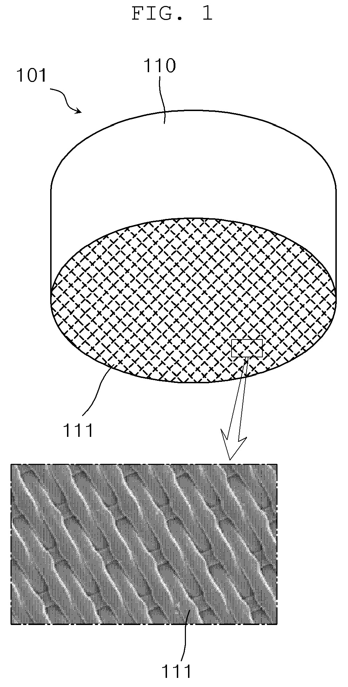

FIG. 1 is an illustration schematically showing a photobioreactor according to a first embodiment of the present invention.

The photobioreactor for mass culturing photosynthetic microorganisms using a mesh sheet according to the present invention may maximize degrees of growth of photosynthetic microorganisms with minimized cost, so that the photosynthetic microorganisms may be efficiently mass produced. In addition, the photobioreactor is placed to float on a water surface or placed underwater to submerge to a certain depth in order to provide an effect of overcoming spatial constraints for mass culture.

As shown in FIG. 1, the photobioreactor 101 according to the first embodiment of the present invention includes a culture container 110 having a barrier to spatially separate environmental water and photosynthetic microorganisms to be cultured, wherein a whole or part of the barrier is prepared with a mesh sheet 111. As the mesh sheet, likewise a perforated sheet, any material, which allows gas, water, and nutrients to freely pass through, while restricts free diffusion of photosynthetic microorganisms, is available.

Particularly, the mesh sheet is characterized by allowing environmental water, gas and nutrients to freely pass through, while restricting free diffusion of photosynthetic microorganisms or contaminant microorganisms. More particularly, since environmental water may be introduced, nutrients required for growth of photosynthetic microorganisms may be supplied, and waste excreted during growth of photosynthetic microorganisms may be removed with environmental water. Since an additional nutrient supplier and purifier is not required, cost, time, and labor-saving effect is provided. Moreover, generated oxygen may be released, and carbon dioxide required for photosynthesis of photosynthetic microorganisms may be supplied through the mesh sheet. Further, since photosynthetic microorganisms are cultured in a manageable and restrictive culture container, it is possible to prevent environmental contamination caused by mass propagation of photosynthetic microorganisms, and to facilitate harvest of mass cultured photosynthetic microorganisms. In particular, the photobioreactor prepared by using the mesh sheet 111 according to the first embodiment of the present invention provides an effect of increasing growth of photosynthetic microorganisms by about 1.5 to 2 times of that of a photobioreactor prepared by using a typical semipermeable membrane, indicating that production efficiency of the photosynthetic microorganisms is significantly improved.

The mesh sheet is characterized by allowing water, nutrients, gas and waste of photosynthetic microorganisms to be freely introduced and released, while blocking free diffusion of the photosynthetic microorganisms. For example, the mesh sheet may be woven with a polymer fabric. The polymer may be a biodegradable polymer or hardly degradable polymer. The biodegradable polymer may be one or at least two selected from the group consisting of polycaprolactone, poly lactic acid, poly(lactic-co-glycolic acid) copolymer, cellulose, methyl cellulose, ethyl cellulose, cellulose acetate, nitrocellulose, curdlan, polyglutamic acid, polylysine, polyhydroxy alkanoate, polyethylen glycol, polyglycolic acid, and polyester, but not limited thereto.

In addition, the hardly degradable polymer may be one or at least two selected from the group consisting of teflon (polytetrafluoroethylene), polyolefine, polyamides, polyacrylate, silicon, poly methyl methacrylate, polystyrene, ethylene-vinyl acetate copolymer, polyethylene-maleic anhydride copolymer, polyamide, poly(vinyl chloride), poly(vinyl fluoride), poly vinyl imidazole, chlorosulphonate polyolefin, polyethylene terephthalate (PET), nylon, low density polyethylene (LDPE), high density polyethylene (HDPE), acryl, polyetherketone, polyimide, polycarbonate, polyurethane, and polyethylene oxide, but not limited thereto.

The culture container prepared by using the mesh sheet may be not particularly limited, but be prepared in any shape, for example circular, oval, cone, or cylindrical shape, provided that the shape is capable of holding photosynthetic microorganisms.

In addition, a whole or part of the barrier of the culture container may be prepared with the mesh sheet, and others are prepared by using non-permeable or semipermeable and transparent or translucent material for maintaining a stereoscopic shape, while floating. For example, when one end of a plastic container holding photosynthetic microorganisms is sealed by using the mesh sheet, the container may float near a surface of seawater due to buoyancy of the plastic container.

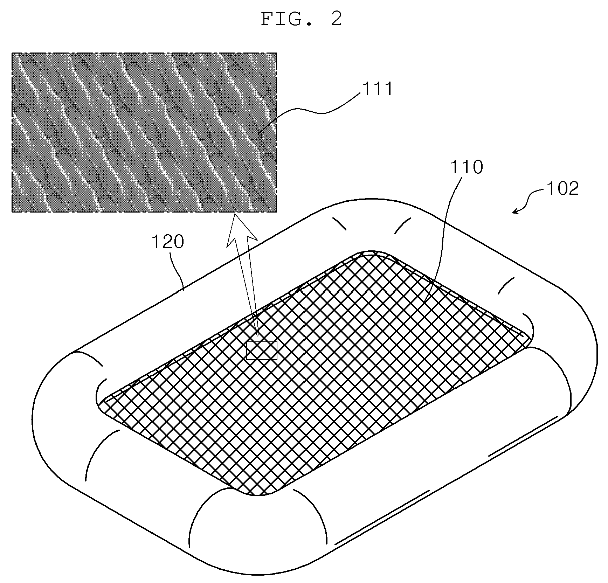

FIG. 2 is an illustration schematically showing a photobioreactor according to a second embodiment of the present invention.

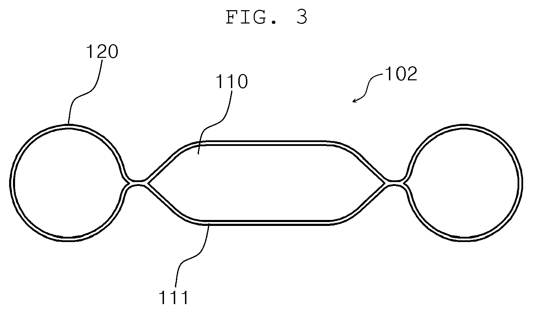

As shown in FIG. 2, the photobioreactor 102 according to the second embodiment of the present invention includes the culture container 110 of the first embodiment, and may further include a buoyant unit 120 in a tube shape extended from the culture container 110. The buoyant unit in a tube shape may be formed with a material the same as or different from a base material of the culture container, and preferably the same material in consideration of a manufacturing process. According to an embodiment of the present invention, the buoyant unit in a tube shape may be formed by: introducing air to form a culture container in a balloon shape; and adhering boundaries thereof through heat-press. In that case, a part of the culture container 110 is surrounded by the buoyant unit 120, and a whole or part of the culture container 110 may be substituted with the mesh sheet 111. FIG. 3 is an illustration showing a cross sectional view of the photobioreactor 102 according to the second embodiment.

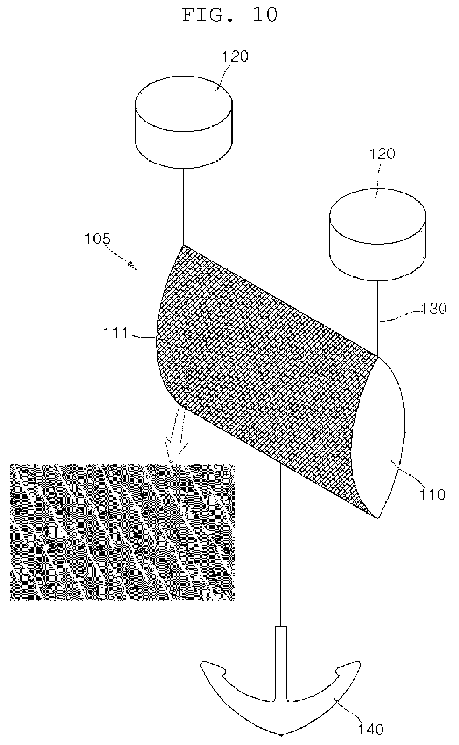

The buoyant unit may be an article such as typical styrofoam, a buoy, and a vacant vessel, coupled to the culture container through a coupling unit (see FIG. 10).

FIG. 3 is an illustration schematically showing a photobioreactor 103 according to a third embodiment of the present invention.

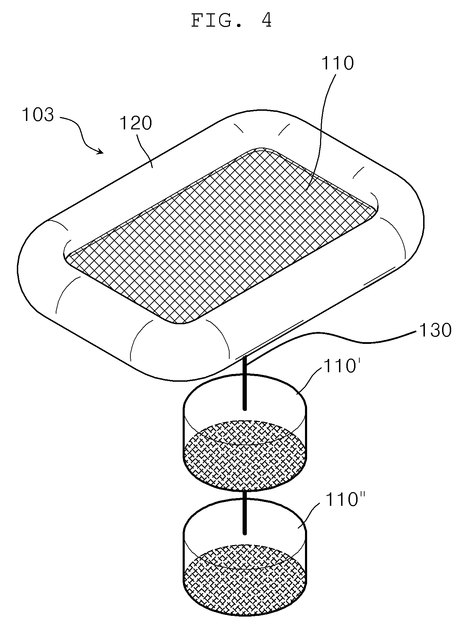

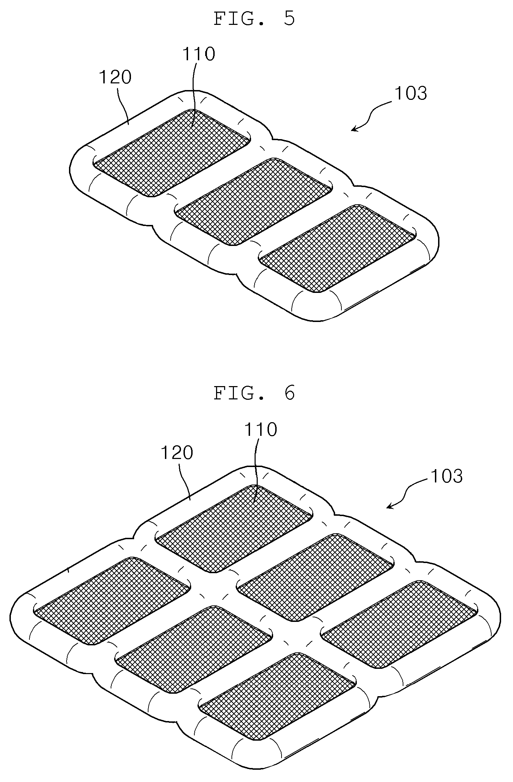

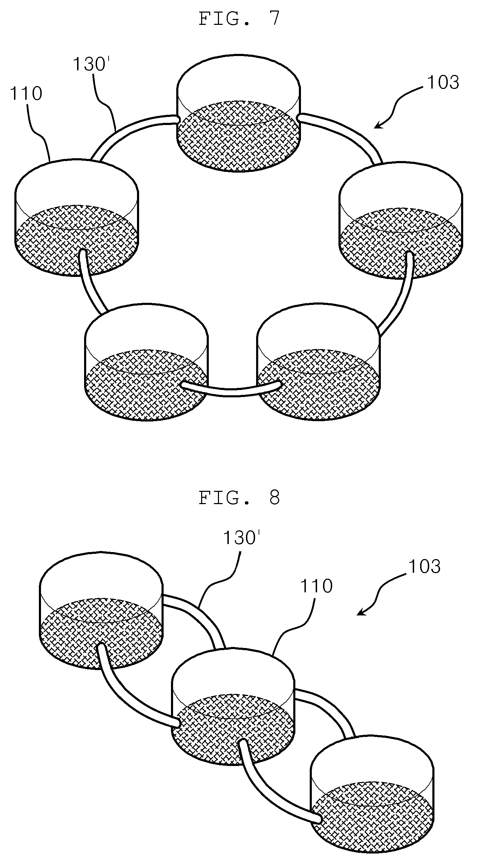

As shown in FIG. 3, the photobioreactor 103 according to the third embodiment of the present invention includes the culture container 110 of the first embodiment, and may further include one or more coupling unit 130 coupled to the culture container 110. The coupling unit 130 may be anyone, regardless of shapes or materials, provided that the coupling unit is capable of coupling the culture containers according to an embodiment of the present invention each other or coupling and securing the culture container and buoyant unit. For example, a ropes or chain may be used. The culture containers 110 may be vertically coupled. In that case, the buoyant unit 120 is only included in the uppermost culture container, and culture containers 110', 110' coupled downward to the uppermost culture container do not include the buoyant unit 120 (FIG. 4). Optionally, the photobioreactor 103 according to an embodiment of the present invention has a plurality of culture containers 110 which may be coupled in series (FIG. 5) or parallel (FIG. 6) by the buoyant units 120 coupled with each other without a separate coupling unit. Optionally, as shown in FIGS. 7 and 8, a plurality of culture containers 110 without a buoyant unit may be coupled in a circular shape (FIG. 7) or in every directions (FIG. 8) by a floating-type coupling unit (130') which is prepared with a material capable of floating (e.g. buoy, styrofoam, or plastic vessel or tube having a vacuum inside or including air or gas therein capable of providing buoyancy) to float the culture containers 110 on a water surface. In that case, the culture container 110 may further include the buoyant unit 120 (not shown). In any cases, types of coupling the culture containers vary depending on types of photosynthetic microorganisms to be cultured and environment of sea where the culture containers are placed. For example, when one or more types of photosynthetic microorganisms are cultured, the culture containers may be vertically coupled and then placed on sea. Additionally, when one type of photosynthetic microorganism is cultured, a floating-type coupling unit having adjusted buoyancy may be used to couple the containers from side to side or in a circular shape such that the containers are located within a depth range of water to which solar light required for culture of the photosynthetic microorganism penetrates.

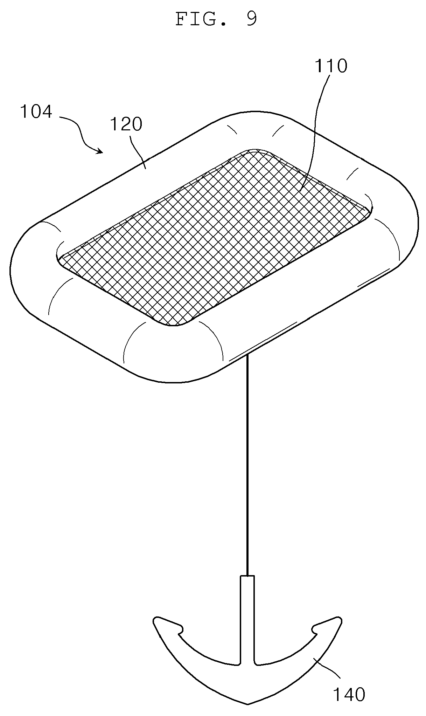

FIG. 9 is an illustration schematically showing a photobioreactor 104 according to a fourth embodiment of the present invention.

As shown in FIG. 9, the photobioreactor 104 according to the fourth embodiment of the present invention includes the culture container 110 of the first embodiment, and may further include one or more sedimentation unit 140 coupled to the culture container 110 so that the culture container 110 submerges to appropriate water depth. The sedimentation unit 140 may be a plumb bob, or a structure placed under the water surface or underwater.

FIG. 10 is an illustration schematically showing a photobioreactor 105 according to a fifth embodiment of the present invention.

As shown in FIG. 10, the photobioreactor 105 according to the fifth embodiment of the present invention includes the culture container 110 according to the first or second embodiment, and may further include a buoyant unit 120 and a sedimentation unit 140 coupled to the culture container 110. For example, the buoyant unit 120 may be coupled to one end of the culture container 110 through a coupling unit 130, and the sedimentation unit 140 may be coupled to the other end of the culture container 110. Through the buoyant unit 120 and the sedimentation unit 140, degrees of floating and sedimentation of the culture container may be adjusted.

FIG. 6 is an illustration schematically showing a photobioreactor 106 according to a sixth embodiment of the present invention.

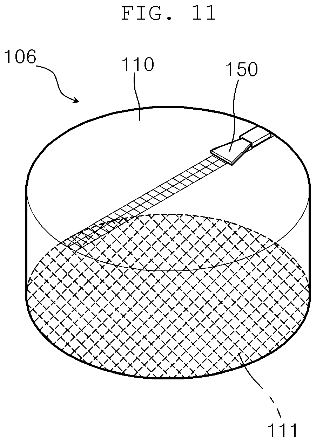

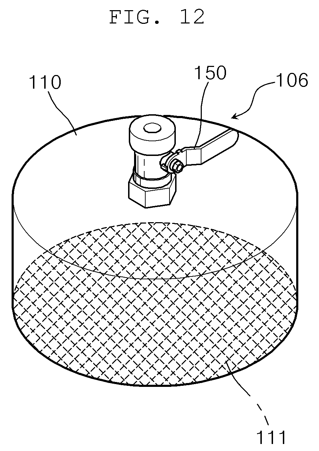

As shown in FIGS. 11 and 12, the photobioreactor 106 according to the sixth embodiment of the present invention includes the culture container 110 according to the first embodiment, and may further include a switching unit 150 coupled to the culture container 110. For example, the switching unit 150 is included in one face of the culture container 110 to allow the culture container 110 to hold photosynthetic microorganisms, wherein the switching unit may have a shape such as a zipper (FIG. 11), a zipper bag or valve (FIG. 12) to facilitate switching.

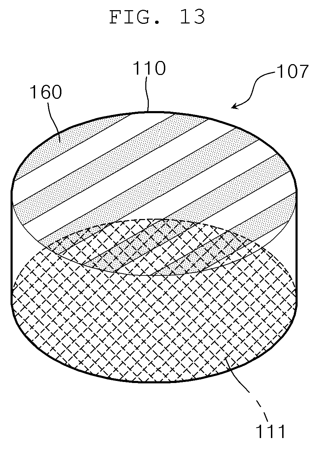

FIG. 13 is an illustration schematically showing a photobioreactor 107 according to a seventh embodiment of the present invention.

As shown in FIG. 13, the photobioreactor 107 according to the seventh embodiment of the present invention includes the culture container 110 according to the first embodiment, and may further include a light blocking area 160 in one face of the culture container 110. For example, by including the light blocking area 160 in one face of the culture container 110, the photobioreactor is configured to adjust wavelengths or amount of light energy delivered to photosynthetic microorganisms to be cultured, wherein the light blocking area may be included in the culture container in a shape such as a pattern.

FIG. 8 is an illustration schematically showing a photobioreactor 108 according to an eighth embodiment of the present invention.

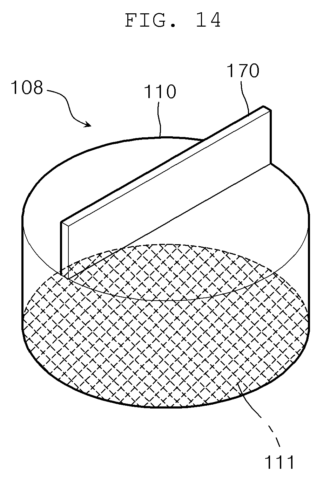

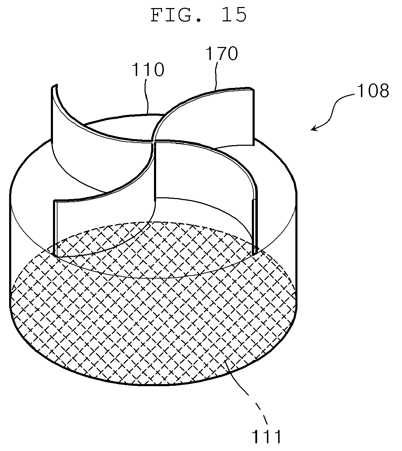

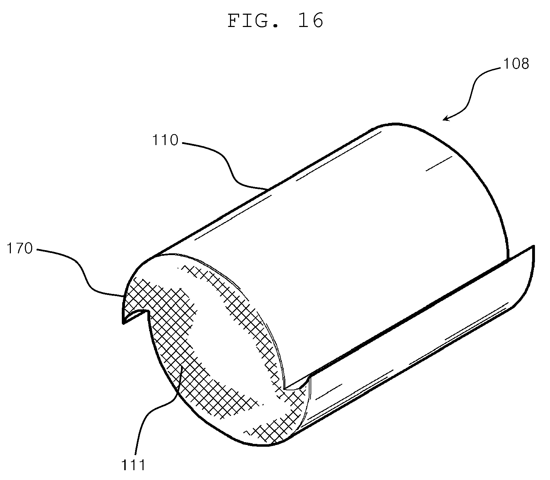

The photobioreactor 108 according to the eighth embodiment of the present invention is characterized by having a wobbling structure allowing a culture container 110 to wobble by wind or wave. For example, as shown in FIG. 14, the culture container 110 according to the first embodiment is included, and a fan 170 coupled to the culture container may be further included in a wobbling structure. The fan 170 is configured to attach a lower or upper face of the culture container to allow the culture container to be rotated in a vertical direction by a force caused by wind or water. One or two crossing fans 70 may be attached to the lower or upper face of the culture container. Also, as shown in FIG. 15, the fan may be configured in a curved shape to broaden a cross section where wind is reached to facilitate rotation. Moreover, the wobbling structure may be applied to a shape of the culture container itself 110. For example, as shown in FIG. 16, both sides of the culture container 110 are elongated, thereby forming the fan 170 similar to a pinwheel, such that the culture container 110 is capable of wobbling by wind or wave. In that case, for efficient wobbling of the culture container 110, both protruded fans 170 are directed in an angle of 180 degrees from each other. Optionally, the wobbling structure may include an outer rotary fan 170', which is attached to an axis 112 penetrating the culture container and rotated by wind or wave, and an inner rotary fan 170" which is interlocked to the outer rotary fan 170' and rotated inside of the culture container. A shape of the outer rotary fan 170' may be any structure such as pinwheels, propellers, or waterwheels, provided that the structure is capable of rotating by wind or flow of water.

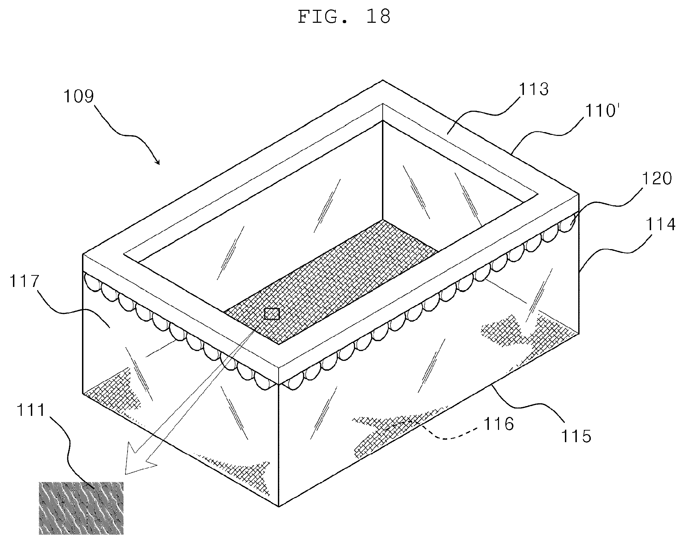

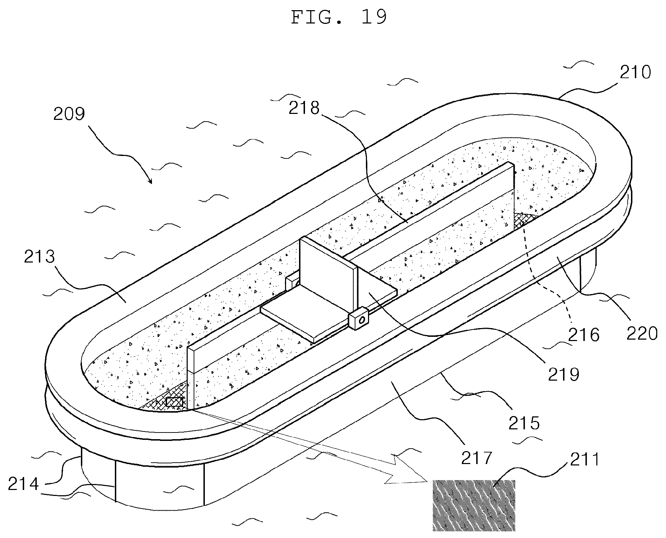

FIGS. 18 and 19 are illustrations schematically showing a photobioreactor 109 according to a ninth embodiment of the present invention. FIG. 18 illustratively shows a cage-type photobioreactor, and FIG. 19 illustratively shows a raceway-type photobioreactor.

As shown in FIGS. 18 and 19, the photobioreactor 109 according to the ninth embodiment of the present invention may have an open-type culture container 110' having an open upper face. In that case, the culture container 110' may include an upper frame 113 and a barrier coupled thereto, wherein a part or whole of the barrier is prepared with a material 111 such as a mesh sheet or perforated sheet which allows gas, water, nutrients to freely pass through, while restricts free diffusion of photosynthetic microorganisms. The upper frame 113 may have any shape such as an oval, a circle, or a polygon, for example a rectangle or square, capable of forming a stereoscopic structure of the open-type culture container 110', and the barrier may be coupled to the upper frame 113 with a shape similar to a scoop net (not shown). Optionally, to maximize a volume of the open-type culture container 110' and stabilize the structure, vertical frames 114 coupled to the upper frame and/or lower frame 115 may be additionally provided. Hereinafter, as shown in FIG. 18, the open-type culture container 110' is described in more detail, wherein the culture container is provided with the rectangular upper frame 113, vertical frames 114 at four edge of the rectangle, lower frame 115 for coupling the vertical frames 114, bottom wall 116 for coupling frames to frames, and four side walls 117 in all directions. A part or whole of the bottom wall 116 may be prepared with the material 111 such as a mesh sheet or perforated sheet which allows gas, water, and nutrients to freely pass through, while restricts only free diffusion of photosynthetic microorganisms, and remaining parts may be prepared with an opaque film material capable of substantially confining the photosynthetic microorganisms. Optionally, the bottom wall 116 may be prepared with an opaque film material and a part or whole of four side walls 117 may be prepared with the material 111 such as a mesh sheet or perforated sheet which allows gas, water, and nutrients to freely pass through, while restricts only free diffusion of photosynthetic microorganisms, or both of the bottom wall 116 and four side walls 117 may be prepared with the material 111 such as a mesh sheet or perforated sheet which allows gas, water, and nutrients to freely pass through, while restricts only free diffusion of photosynthetic microorganisms. The open-type culture container 110' having such structure, however, may have difficulty in floating onto a water surface, and thus a part or whole of the upper frame 113 may be provide with a separate buoyant unit 120. Optionally, the open-type culture container 110' may have a structure in which the upper frame itself 113 is prepared with a buoyant material or the upper frame 113 is directly and indirectly coupled to the buoyant unit 120. In the former case, the upper frame 113 of the culture container 110' may be prepared as one or more tube into which air is introduced, or a vacant plastic frame. In the latter case, a buoyant unit (e.g. styrofoam, or a plastic vessel having a vacuum inside or including air or gas capable of providing buoyancy) may be attached to a side or lower part of the upper frame 113. A workbench may be added to the upper frame 113 to facilitate a process of inoculating or collecting photosynthetic microorganisms by an operator. Optionally, the workbench may be omitted in the case where the upper frame itself 113 directly plays a role as a buoyant unit or the upper frame 113 is overlaid with the buoyant unit 120. As necessary, the upper frame 113 may house the bottom wall 116 or four side walls 117 to adjust a depth of the culture container 110'. In other word, the upper frame 113 may house the bottom wall 116 or four side walls 117 under cloudy weather or season of weak solar light such that a depth of the culture container 110' may become smaller, whereas the bottom wall 116 or four side walls 117 protrude from the upper frame 113 under sunny weather or season of strong solar light such that a depth of the culture container 110' may become greater.

Moreover, as shown in FIG. 19, a photobioreactor 209 according to another embodiment of the present invention may include an open-type culture container having a raceway pond 210 placed to float on a water surface. In that case, as shown in FIG. 19, a partition 218 may be placed on middle of the open-type culture container having a raceway pond 210 such that the culture container has a structure in which a culture medium may be rotated in one direction. In that case, for smooth rotation of the culture medium, a culture medium circulator 219 such as a waterwheel or pump may additionally be included. Basically, the culture medium circulator 219 may be rotated by wind or wave, and additionally be provided with a motor supplying rotational force by a separate power such as batteries or photovoltaic power generators for continuous rotation in one direction (not shown). A basic structure of the open-type culture container having a raceway pond 210 is similar to that of the open-type culture container 110' illustrated in FIG. 18. The open-type culture container having a raceway pond 210 may include an upper frame 213, and a barrier coupled to the upper frame 213, wherein the upper frame may be formed in a oval, longitudinally elongated oval, or circular shape. To maximize a volume of the open-type culture container having a raceway pond 210 and stabilize the structure, the container may further be provided with vertical frames 214 coupled to the upper frame 213 and/or a lower frame 215. Hereinafter, as shown in FIG. 19, the open-type culture container having a raceway pond 210 is described in more detail, wherein the culture container includes the upper frame 213 in a longitudinally elongated oval shape, vertical frames 214 coupled downward to the oval-shaped upper frame 213, the lower frame 215 coupling the vertical frames 214, side walls 217 and bottom wall 216 for coupling the frames. A part or whole of the bottom wall 216 may be prepared with a material 211 such as a mesh sheet or perforated sheet which allows gas, water, and nutrients to freely pass through, while only restricts free diffusion of photosynthetic microorganisms, and remaining parts may be prepared with an opaque film material capable of substantially confining photosynthetic microorganisms. Optionally, the bottom wall 216 may be prepared with an opaque film material and a part or whole of side walls 217 may be prepared with the material 211 such as a mesh sheet or perforated sheet which allows gas, water, and nutrients to freely pass through, while restricts only free diffusion of photosynthetic microorganisms, or both of bottom wall 216 and side walls 217 may be prepared with the material 211 such as a mesh sheet or perforated sheet which allows gas, water, and nutrients to freely pass through, while restricts only free diffusion of photosynthetic microorganisms. The open-type culture container having a raceway pond 210 in such structure, however, may have a difficulty in floating onto a water surface, and thus a part or whole of the upper frame 213 may be provide with a separate buoyant unit 220. Optionally, the open-type culture container having a raceway pond 210 may have a structure in which the upper frame itself 213 is prepared with a buoyant material or the upper frame 213 is directly overlaid with the buoyant unit 220. In the former case, the upper frame 213 of the culture container having a raceway pond 210 may be prepared as one or more tube into which air is introduced or a vacant plastic frame. A workbench may be added to the upper frame 213 to for facilitate a process of inoculating or collecting photosynthetic microorganisms by an operator. Optionally, the workbench may be omitted in the case where the upper frame itself 213 directly plays a role as a buoyant unit, or the upper frame 213 is overlaid with the buoyant unit 220.

As above, when the culture container having the open upper part, and a barrier, which is placed at a whole or part of the side and/or bottom face, allowing gas, water, and nutrients to freely pass through, while restricting free diffusion of photosynthetic microorganisms is used, it is possible to more efficiently culture photosynthetic microorganisms because the raw cost is greatly reduced; gases are more freely exchanged; waste generated during photosynthesis are readily removed; and nutrients are readily supplied from environmental water.

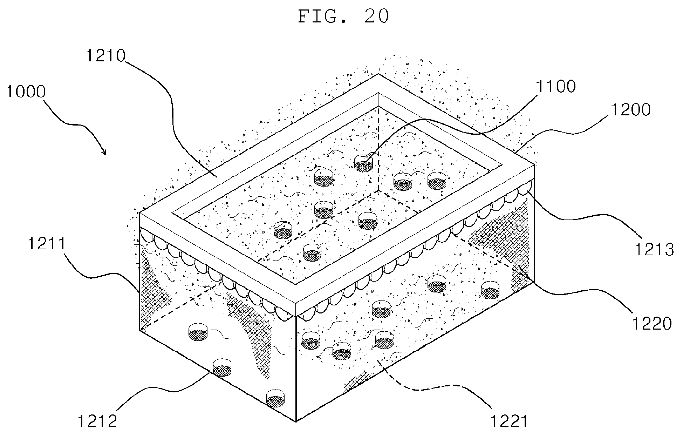

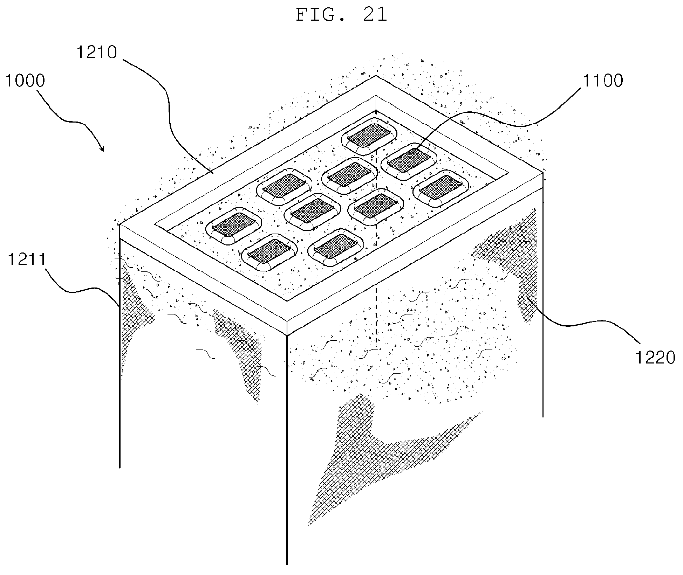

FIGS. 20 and 21 are illustrations schematically showing a culture facility for photosynthetic microorganisms according to an embodiment of the present invention. FIG. 20 illustratively shows a culture facility including a photobioreactor without a buoyant unit, and FIG. 21 illustratively shows a culturing filed including a photobioreactor provided with a buoyant unit.

A culture facility for photosynthetic microorganisms 1000 according to an embodiment of the present invention may include a photobioreactor 1100 and a floating structure 1200 holding the photobioreactor, wherein, in the culture facility, a plurality of culture containers capable of culturing photosynthetic microorganisms are secured at a certain location of a water surface to facilitate collection after culture without loss. To prevent loss of the photobioreactor 1100, the floating structure 1200 includes an upper frame of culture facility 1210 and fences 1220 for coupling the upper frames of curling field 1210 to separate inside from outside of the culture facility 1000. As shown in FIG. 20, in addition to the upper frame of culturing filed 1210 floated on a water surface, the culture facility for photosynthetic microorganisms 1000 according to an embodiment of the present invention may further include vertical frames of culturing filed 2011 and a lower frame of culture facility 1212 which submerge under the water surface, wherein the upper frame of culture facility 1210 may have any shape such as an oval, circle, or polygonal, for example rectangle, or square, capable of stereoscopically forming a structure of a culture container 1110, and in the case where fences 1220 are coupled to the upper frame of culturing filed 1210 like a scoop net (not shown), the vertical frames of culture facility 1211 and the lower frame of culturing filed 1212 may be omitted. The fences 1220 may be prepared with various materials such as plastics, wood, plywood, nets, and preferably nets in consideration of costs and free communication of environmental water. The culture facility for photosynthetic microorganisms 1000 thus formed has a structure similar to a sort of floating fish cages. The upper frame of culture facility 1210 of the floating structure 1200 on a water surface may be provided with a buoyant unit of culture facility 1213 to float the floating structure 1200 onto a water surface. The floating structure of culturing filed 1213 may adjust buoyancy taken into account conditions such as solar light energy and nutritional salts required for culture of photosynthetic microorganisms to be cultured. The buoyant unit of culture facility 1213 may be prepared with various materials such as styrofoam, and a vacant plastic vessel, and may overlay the upper frame of culturing filed 1210 or be coupled to the upper frame of culture facility 1210 with a separate coupling unit. In addition, the floating structure 1200 may be configured to include an operator supporting unit (not shown) allowing an operator to conduct a management work. The operator supporting unit plays a role as a support on which an operator may conduct a work, and also the operator supporting unit may be coupled to the buoyant unit of culture facility 1213 or to an underwater or floating facility separated from the buoyant unit of culturing filed 1213 (not shown). The operator supporting unit itself may be prepared with a buoyant material, thereby replacing the buoyant unit of culturing filed 1213. Namely, when the supporting unit (not shown) prepared with a buoyant material overlays the upper frame of culturing filed 1210, the buoyant unit of culturing filed 1213 may be omitted. As shown in FIG. 21, when the upper frame of culture facility 1210 is prepared with a buoyant material (e.g. tube or vacant plastic frame), the buoyant unit of culturing filed 1213 and operator supporting unit may be omitted. The photoreactor 1000 may be provided with or without a buoyant unit. When the photobioreactor 1000 is not provided with a buoyant unit, a depth of a bottom face 1221 of the floating structure 1200 may be adjusted to prevent the photobioreactor 1000 from submerging under the water surface too deep. Merely, when the photobioreactor 1100 is provided with a buoyant unit, as shown in FIG. 21, the bottom face 1221 and lower frame of culture facility 1212 of the floating structure 1200 may be omitted.

As above, when culture is performed by confining the photobioreactor according to an embodiment of the present invention by using the culturing filed of photosynthetic microorganisms, it is possible to prevent loss of the photobioreactor without a particular securer, and to facilitate putting and collecting of the photobioreactor.

### MODE FOR CARRYING OUT THE INVENTION

Hereinafter, the present invention will be described in more detail with reference to experimental examples. The present invention may, however, be embodied in various forms differs from each other, and should not be construed as limited to the experimental examples set forth hereinafter, the experimental examples are provided so that the disclosure of the present invention will be complete, and will fully convey the scope of the present invention to those skilled in the art.

### EXPERIMENTAL EXAMPLE 1: MEASUREMENT OF PHOTOSYNTHETIC MARINE MICROORGANISM PERMEABILITY OF PHOTOBIOREACTOR PREPARED WITH MESH SHEET

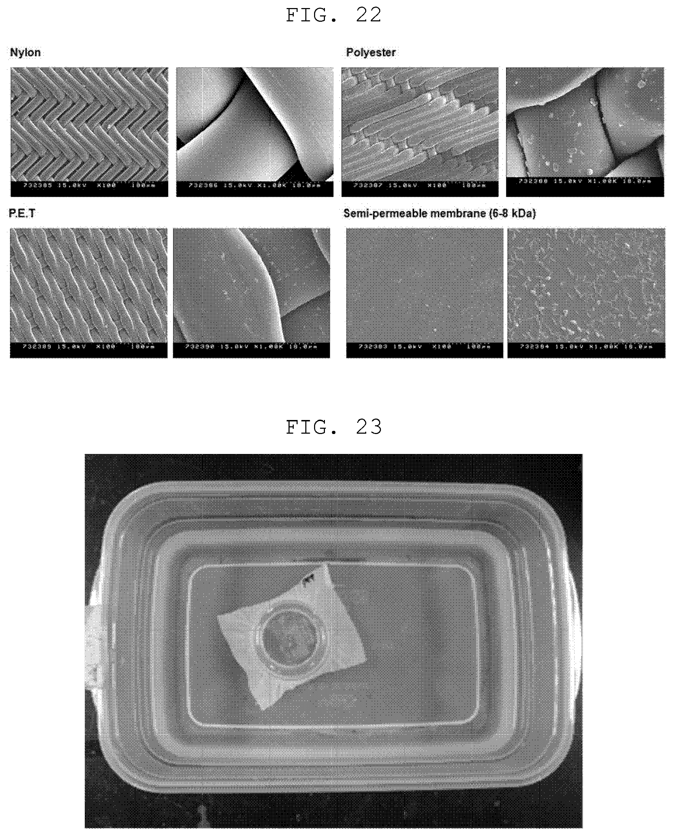

An experiment was conducted to investigate whether photosynthetic marine microorganisms are released through a mesh sheet. Culture containers were constructed by attaching mesh sheets respectively prepared with PET, polyester and nylon to bottoms of plastic containers having a total volume of 100 ml. 60 ml of photosynthetic microorganism (wet weight: 0.5 g/L) (*Tetraselmis* sp.) was placed into the culture container and the container was allowed to float on a plastic container containing 1 L of f/2-Si medium to investigate whether the photosynthetic microorganisms were released to outside of the culture containers prepared with the mesh sheets. An opening size for nylon and PET was 5 µm, and an opening size for polyester was 15 µm. If photosynthetic microorganisms are released to outside of the mesh sheet, the photosynthetic microorganisms would grow using a medium at outside. During culture of photosynthetic microorganisms, temperature was maintained at 20□, and 100 µE/m²/s of intensity of light was supplied using fluorescent light. After five days of culture, concentrations of photosynthetic microorganisms at inside and outside of the reactor were measured with coulter counter (model: multi-sizer 3, Beckman Inc., USA). FIG. 22 shows scanning electron microscopy results of nylon, polyester, PET mesh sheet and a semipermeable membrane having the molecular weight cut-off value of 6-8 kDa used in the experiment.

Characterization of the mesh sheet used and volume of culture medium contained in the culture container set forth in Table 1 below.

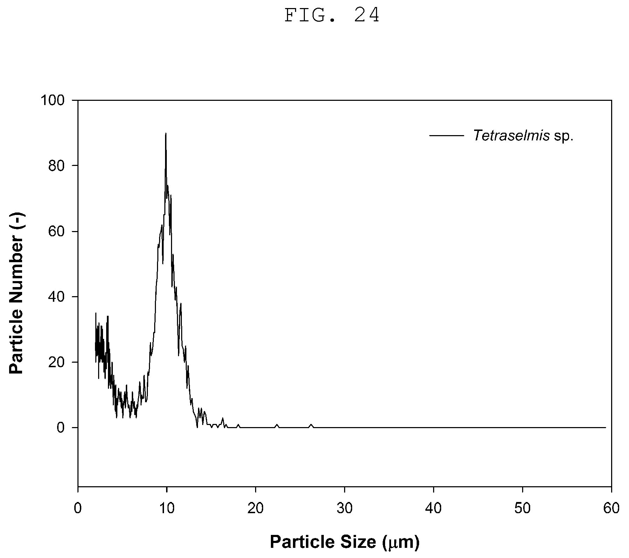

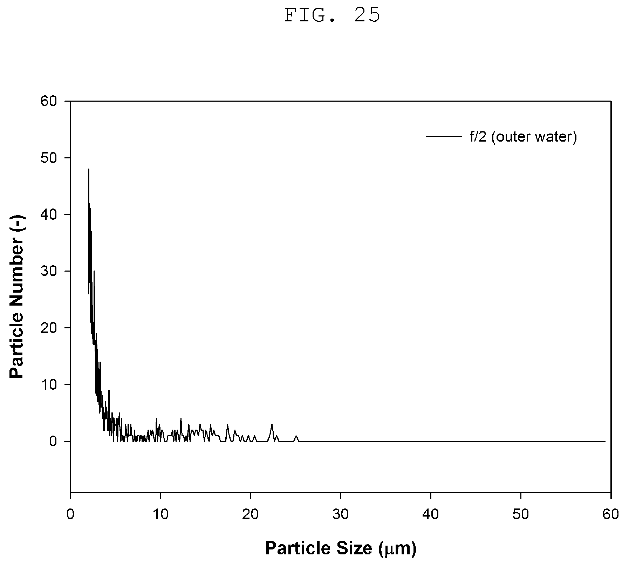

As shown in FIG. 23, one face of the plastic container was sealed with the mesh sheet using PET, and photosynthetic microorganisms were introduced into the container and cultured to investigate whether the photosynthetic microorganisms are released or not. Consequently, it can be found that cell size and dispersity of the photosynthetic microorganisms in the culture container were exhibited as shown in FIG. 24, and that photosynthetic microorganisms having the average size of about 11 µm were dispersed in the culture container. Also, as shown in FIG. 25, it can be found that although cells having a size of about 5 µm or less were observed at outside of the culture container, cultured photosynthetic microorganisms were not released from the culture container (see FIGS. 24 and 25).

**[Table 1]**

| | Length (m) | Width (m) | Surface area (m²) | Thickness (m) | Volume of culture medium (m³) |
|---|---|---|---|---|---|
| Nylon | 0.065 | 0.045 | 0.002925 | 0.000100 | 0.00006 |
| Polyester | 0.065 | 0.045 | 0.002925 | 0.000065 | 0.00006 |
| PET | 0.065 | 0.045 | 0.002925 | 0.000033 | 0.00006 |

### EXPERIMENTAL EXAMPLE 2 : MEASUREMENT OF DEGREE OF GROWTH OF PHOTOSYNTHETIC MICROORGANISMS USING PHOTOBIOREACTOR PREPARED WITH MESH SHEET

Photosynthetic microorganisms were cultured by practically using the culture container prepared with the mesh sheet, and also photosynthetic microorganisms were cultured by using a semipermeable membrane of a cellulose material as a control.

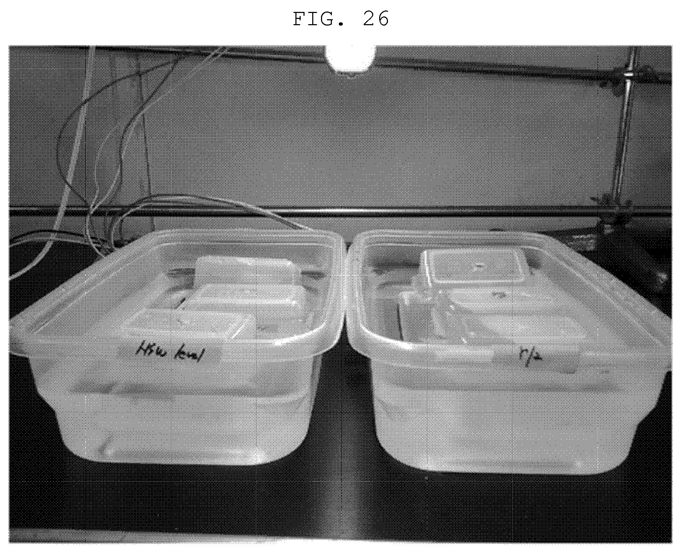

1 L of f/2-Si, and 1 L of near seawater (NSW) were poured into two plastic water baths having a capacity of containing 2 L of an aqueous solution, wherein the NSW was prepared by diluting f/2-Si medium to 1/30 in order to adjust nitrogen and phosphorus concentration similar to that of dissolved in seawater of Incheon. The mesh sheet with polyester or nylon material (0.003 m²) was attached to a bottom of the plastic reactor. 100 ml of culture medium, to which 0.05 g/l (wet weight) of photosynthetic microorganisms were inoculated, was poured into the plastic container, and then the container was allowed to float on the water bath while photosynthetic microorganisms were cultured (FIG. 26). As a control, the same width of semipermeable membrane of a cellulose material having 50 kDa of molecular weight cut-off was attached, and then experiment was conducted to compare degrees of growth of photosynthetic microorganisms. To maintain a concentration difference of nutritional salts between inside and outside of the culture container, the medium was periodically replaced once a day. 100 µE/m²/s was supplied for 24 hours by using fluorescent light during culture. The culture temperature was maintained at 20□. Culture was continued for 18 days. On 0, 3, 6, 9, 12, 15 and 18 days after culture, 1-2 ml of culture medium was taken through a sampling port placed on an upper part of the culture container to measure degrees of growth of cells and nitrogen concentration of culture medium.

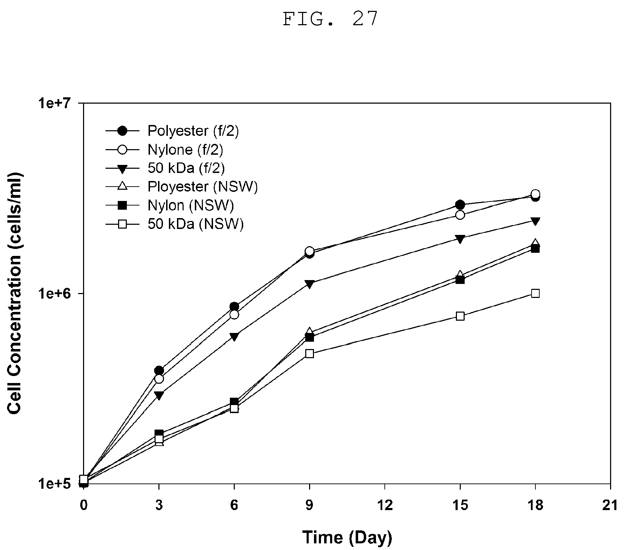

Consequently, as shown in FIG. 27, it can be found that, irrespective of types of medium, growth of photosynthetic microorganisms were better when polyester or nylon mesh sheet was used than when cellulose semipermeable membrane, i.e. a control, was used. Particularly, it has been shown that, in wet weight of photosynthetic microorganisms, a degree of growth was higher by about 25% for microorganisms cultured in f/2 and a degree of growth was higher by about 60-80% for microorganisms in the water bath containing NSW with respect to that of microorganism when 50 kDa cellulose semipermeable membrane, i.e. a control was used (see FIG. 27). These results demonstrate that nutritional salts in outside of the culture container easily penetrate the mesh sheet than semipermeable membrane, and thus the mesh sheet may facilitate growth of photosynthetic microorganisms.

### EXPERIMENTAL EXAMPLE 3: MEASUREMENT OF SALT PERMEABILITY OF PHOTOBIOREACTOR PREPARED WITH MESH SHEET

Then, the present inventor measured nutritional salt permeability of the culture container prepared with the mesh sheet. For a control, a semipermeable membrane of a cellulose material was used to culture photosynthetic microorganisms.

Nutritional salts permeability was measured with a transfer rate of nitrates, which is an important factor for culture of photosynthetic microorganisms. A method for measuring nutritional salt permeability of the mesh sheet was as follows: 2 L of seawater including nitrates having concentrations of 100, 200 or 400 mg/L was prepared in a rectangular water bath; a rectangular plastic container containing 100 ml of seawater without nitrates was allowed to float on the water bath; and changes in the concentration difference due to introduction of nitrates from the water bath to the plastic container was measured with lapse of time.

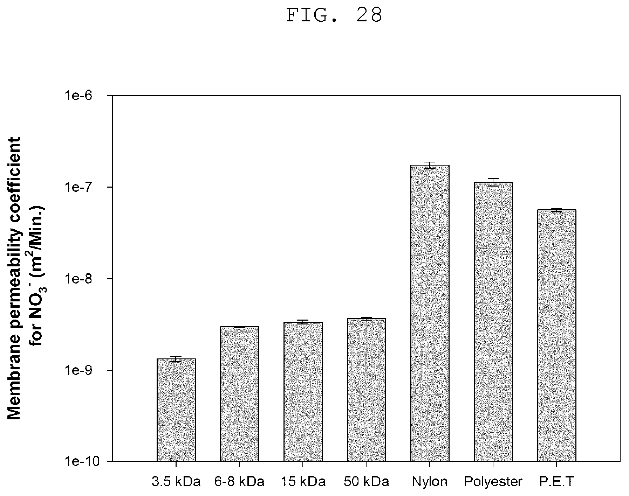

Consequently, as shown in Table 2 below and FIG. 28, it can be found that the nitrate compound penetrates into the culture container through the mesh sheets with nylon, polyester, or PET material about 30 times more easier with respect to cellulose semipermeable membrane. Such trend was not equally exhibited for the semipermeable membrane. Even in the case where molecular weight cut-off was increased to 3.5 kDa, 6-8 kDa, 15 kDa and 50 kDa, amounts of penetration were not increased, and the maximum value was merely about 1/28 of that of the mesh sheet. These results demonstrate that there is limitation in directly culturing photosynthetic microorganisms in environmental water by using the semipermeable membrane alone, and that the culture container prepared with the mesh sheet according to an embodiment of the present invention may provide photosynthetic microorganisms with nutritional salts required for growth without release of photosynthetic microorganisms, thereby efficiently increasing productivity of photosynthetic microorganisms.

**[Table 2]**

| | NO₃-transfer rate (mg/l/min) | Estimated Max. NO₃-transfer rate (mg/m²/day) | Estimated Max. NO₃-transfer rate (g/m²/day) |
|---|---|---|---|
| 3.5 kDa Semipermeable membrane | 0.0291 | 154 | 0.154 |
| 6-8 kDa Semipermeable membrane | 0.0257 | 136 | 0.136 |
| 15 kDa Semipermeable membrane | 0.0325 | 172 | 0.172 |
| 50 kDa Semipermeable membrane | 0.0352 | 187 | 0.187 |
| Nylon mesh sheet | 0.1866 | 5512 | 5.512 |
| Polyester mesh sheet | 0.1869 | 5522 | 5.522 |
| P.E.T mesh sheet | 0.1835 | 5420 | 5.420 |

### EXPERIMENTAL EXAMPLE 4 : MEASUREMENT OF DEGREE OF GROWTH OF PHOTOSYNTHETIC MICROORGANISMS USING PHOTOBIOREACTOR PREPARED WITH MESH SHEET FOR MARINE CULTURE

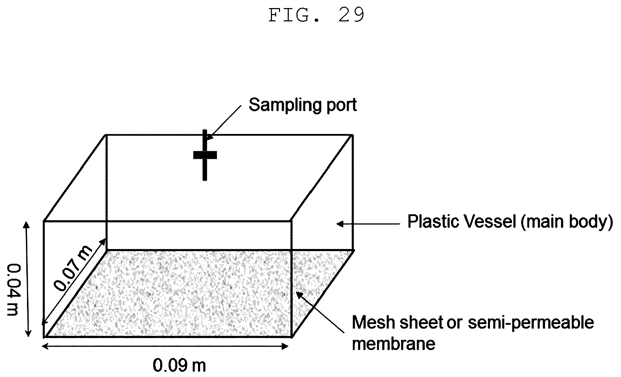

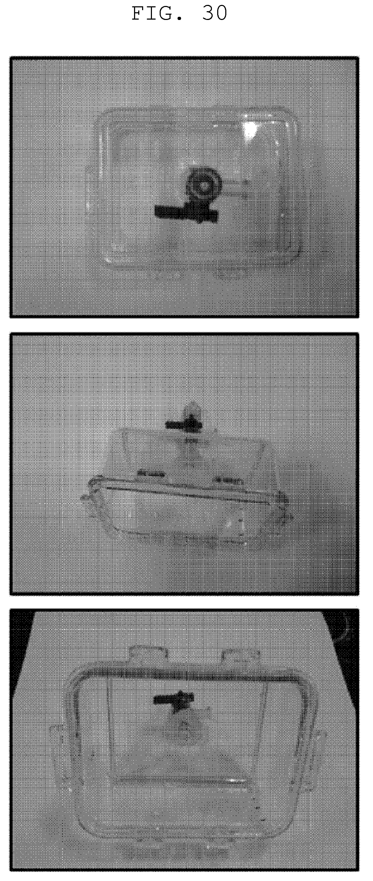

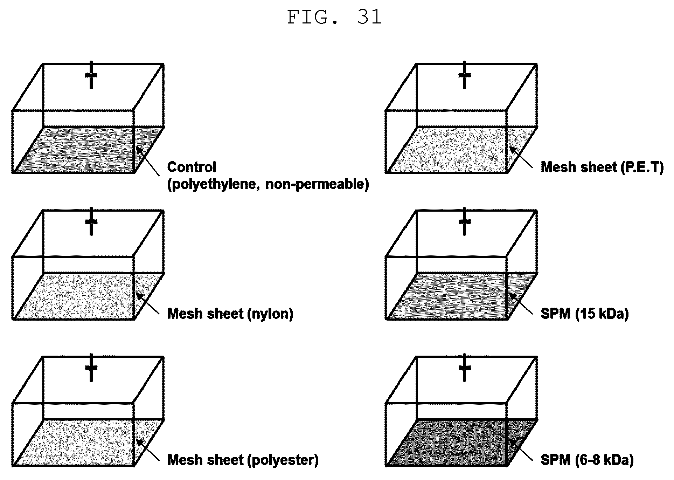

The present inventors placed the photobioreactor according to an embodiment of the present invention on sea culturing filed in Yeongheung-Do. Then, *Tetraselmis sp.* (KCTC12236BP) was cultured for 9 days to investigate growth of the strain and degrees of penetration of a nitrogen source. The same type of photosynthetic microorganisms were cultured in photobioreactors prepared by using non-permeable membrane (polyethylene) and semipermeable membrane together with the reactor prepared with the mesh sheet according to an embodiment of the present invention. The photobioreactor had a structure as shown in FIG. 29. A plastic container as described in FIG. 30 was used. The mesh sheet, non-permeable or semipermeable membrane was applied to a bottom for comparison. Photosynthetic microorganisms were introduced into the culture container (see FIGS. 30 and 31). Particularly, for the non-permeable membrane, polyethylene was used, and, for the mesh sheets, PET, nylon, and polyester were used. Culture was performed by adding three times of f/2-Si medium and 4 g/L of sodium bicarbonate for carbon source supply. As the semipermeable membrane (SPM), semipermeable membranes with a cellulose material having molecular weight cut-off of 6-8 kDa and 15 kDa (product number 132544, spectrumlabs, USA) were used (FIG. 31).

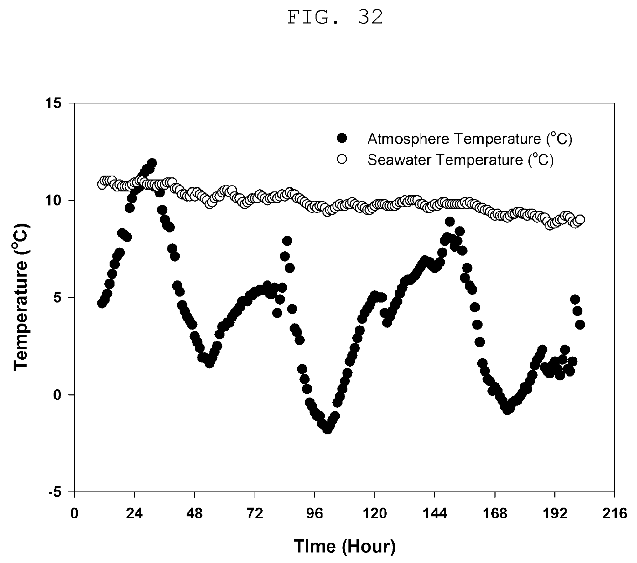

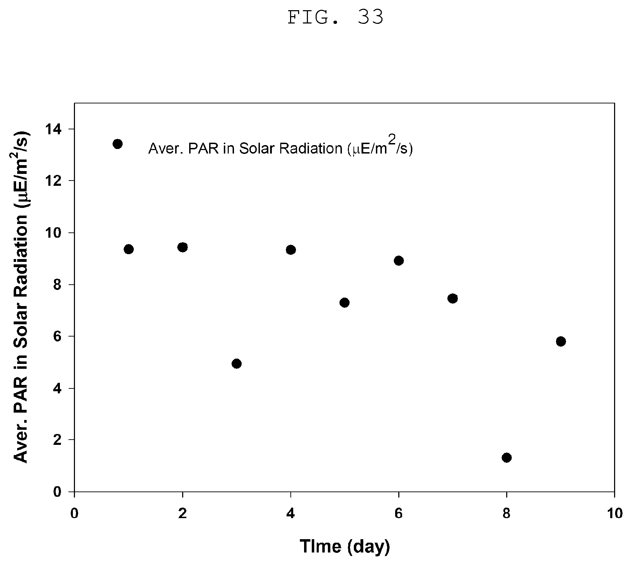

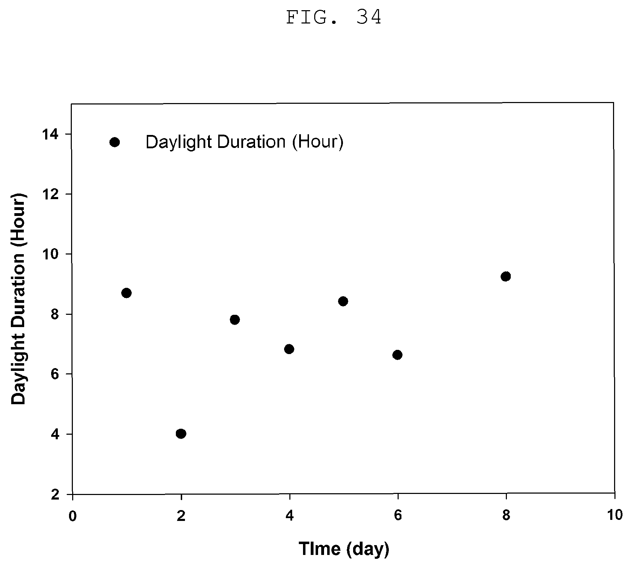

During culturing *Tetraselmis* sp. for 9 days, temperature, water temperature, measured water temperature, and measured temperature of see culture facility for Yeongheung-Do are recorded as in FIG. 32. A day of initiating culture of photosynthetic microorganisms was determined as time 0. In addition, daily photosynthetically active radiation (PAR) and time of light irradiation were summarized in FIGS. 33 and 34. To sum up, during culture of photosynthetic microorganisms, average water temperature was 9.9□; average temperature was 4.2□; average PAR was 295.5 µE/m²/s; and average light irradiation time was 7.4 hours (see FIGS. 32 to 34).

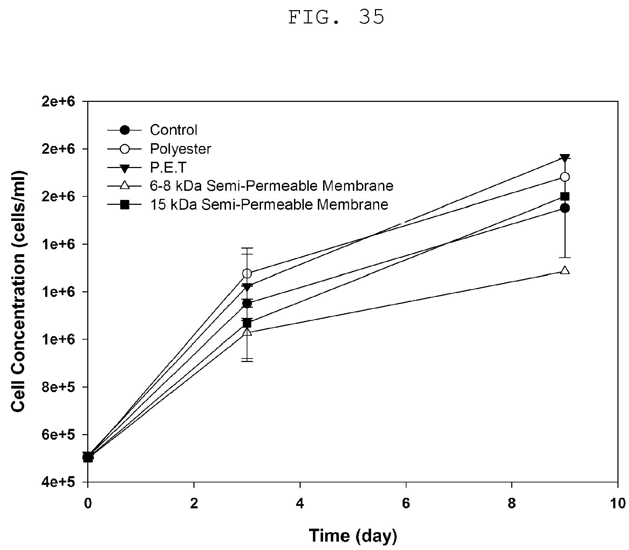

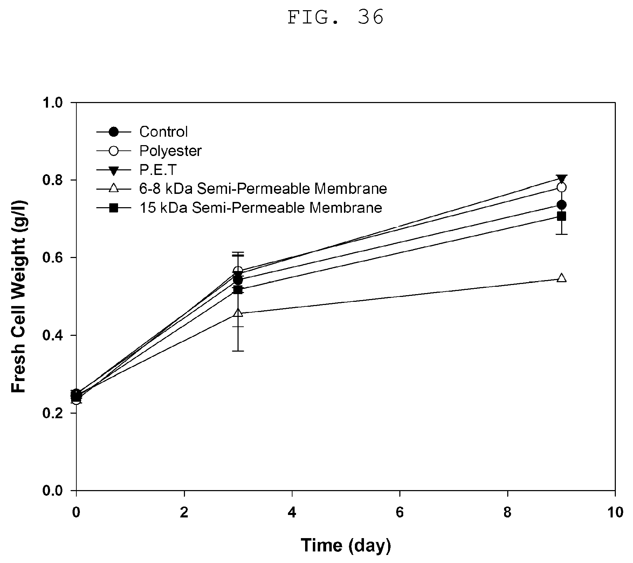

Degrees of growth of photosynthetic microorganisms in the photobioreactor were determined based on cell concentration and wet weight of photosynthetic microorganisms. When the photobioreactors prepared by using the mesh sheets of polyester and PET material were used, growth of photosynthetic microorganisms were respectively increased to 1.68 X 10⁶, and 1.77 X 10⁶ cells/ml (FIG. 35). Also, when the mesh sheets of polyester and PET material were used, wet weights were increased by 1.14-1.48 times of that of the semipermeable membrane of 15 kDa was used (FIG. 36). Interestingly, the semipermeable membrane having a low cut-off value of 6-8 kDa showed the lowest value which was lower than that of the control in which non-permeable membrane was used (see Table 3).

**[Table 3]**

| day 9 | control | Polyester | P.E.T | 6-8 kDa | 15 kDa |
|---|---|---|---|---|---|
| Wet weight (g/l) | 0.79 | 0.78 | 0.81 | 0.55 | 0.71 |
| Cell concentration (cells/ml) | 1.70E+06 | 1.68E+06 | 1.77E+06 | 1.29E+06 | 1.60E+06 |

### EXPERIMENTAL EXAMPLE 5: MEASUREMENT OF REUSE EFFICIENCY OF CULTURE CONTAINER PREPARED WITH MESH SHEET

To investigate reuse efficiency of the mesh sheet according to an embodiment of the present invention, the mesh sheet used in Experimental Example 4 was collected. The mesh sheet was, then, washed or not washed and used to culture *Tetraselmis* sp. strain for 9 days in a culturing device in sea at Yeongheung-Do, while measuring nitrate transfer efficiency. As a control, the semipermeable membranes of a cellulose material having molecular weight cut-off of 6-8 kDa and 15 kDa, which were used in Experimental Example 4, were used after washing or without washing. Also, an unused membrane was used as a control for the whole experiment. Particularly, washing was performed with running tap water as follows: each used membrane was immersed in a 2 L of water bath filled with 1 L of tap water for about five minutes; and the front and reverse sides of the membrane were manually washed with running tap water for about 1 minute without an additional washing tool.

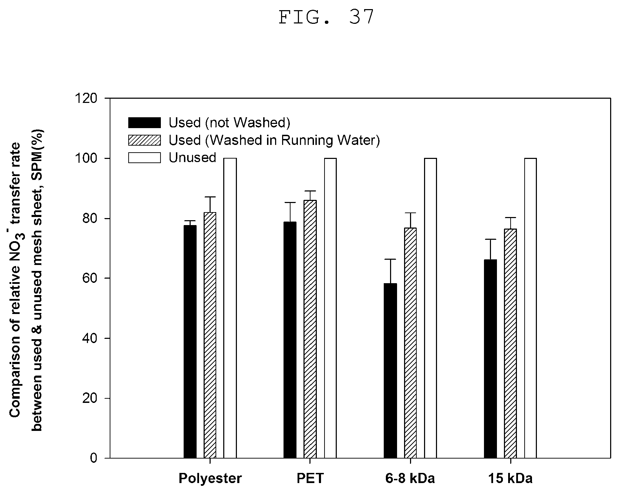

While culturing the *Tetraselmis* sp. strain for 9 days, transfer rates of nitrates through the membranes were compared by the same method as Experimental Example 3. Consequently, as shown in FIG. 37, nitrate penetration efficiency of the photobioreactors prepared with reused polyester and PET mesh sheets was better relative to that of the semipermeable membrane, and also penetration efficiency was increased by simply washing with tap water without an additional process. These results indicates that the mesh sheet according to an embodiment of the present invention may be reused after passing through a simple process of washing with tap water, thereby having excellent industrial availability.

The present invention has been described with reference to the embodiments and experimental examples illustrated in the drawings for only exemplary purpose, and it should be understood that numerous modifications and other equivalent embodiment can be made thereto by a person skilled in the art. Therefore, the technical protection scope of the present invention should be defined by the technical sprit of the appended claims.

### INDUSTRIAL APPLICABILITY

The photobioreactor according to an embodiment of the present invention is allowed to culture of photosynthetic microorganisms with low costs and high efficiency, and can thus be applied to production of biodiesel and useful products such as astaxanthin derived from microalgae, as well as production of foods using microalgae.

## Claims

1. A photobioreactor, comprising a shape-maintainable culture container capable of holding a photosynthetic microorganism to be cultured and liquid for dispersing the photosynthetic microorganisms, wherein a whole or at least a part of the culture container has a barrier which allows water, gas, and nutrients to freely pass through, but restricts diffusion of the photosynthetic microorganisms.

2. The photobioreactor of claim 1, wherein the barrier is a mesh sheet or perforated sheet.

3. The photobioreactor of claim 2, wherein the mesh sheet has a fabric structure.

4. The photobioreactor of claim 2, wherein the mesh sheet has an opening size of 0.1 µm to 200 µm.

5. The photobioreactor of claim 2, wherein the mesh sheet is woven with a polymer fiber.

6. The photobioreactor of claim 5, wherein the polymer is a biodegradable polymer or hardly degradable polymer.

7. The photobioreactor of claim 2, wherein, except the mesh sheet or perforated sheet, remaining parts of the culture container is prepared with a semipermeable or non-permeable and transparent or translucent material.

8. The photobioreactor of claim 2, wherein the perforated sheet is prepared by perforating microholes having a size of between 0.1 to 200 µm in a non-permeable polymer membrane, semipermeable polymer membrane or a mixture polymer membrane thereof.

9. The photobioreactor of claim 1, wherein the culture container is provided with one or more of inlets.

10. The photobioreactor of claim 9, wherein the inlet is provided with a switching unit in a form of a zipper, a zipper bag, a valve, a tub cap, an adhesive tape, a clip, or a clawclip.

11. The photobioreactor of claim 1, wherein the culture container is configured to float on a water surface by a buoyant unit or to submerge under the water surface to a certain depth by a sedimentation unit.

12. The photobioreactor of claim 11, wherein the buoyant unit is in a shape of an air injection tube extended from the culture container.

13. The photobioreactor of claim 11, wherein the sedimentation unit is a plumb bob coupled to a lower part of the culture container, or is an underwater structure placed underwater or under the water surface to allow the culture container to submerge under the water surface in a certain depth.

14. The photobioreactor of claim 11, wherein one end of the culture container is coupled to the buoyant unit and another end of the culture container is coupled to the sedimentation unit.

15. The photobioreactor of claim 11, wherein the culture container is an open-type culture container having an opened upper part.

16. The photobioreactor of claim 15, wherein the open-type culture container has a raceway pond.

17. The photobioreactor of claim 1, wherein one face of the culture container is configured to modulate light energy supplied to the photosynthetic microorganism by a light blocking area.

18. The photobioreactor of claim 1, wherein the culture container is configured to be rotated in a vertical direction by a force from water or wind through a fan attached to a lower or upper face of the culture container.

19. A culture facility for photosynthetic microorganisms, comprising a floating structure comprising the photobioreactor of any one of claims 1 to 18.

20. The culture facility for photosynthetic microorganisms of claim 19, wherein the floating structure comprises a plurality of frame coupled with each other, and fences coupled to the frames.

21. The culture facility for photosynthetic microorganisms of claim 19, wherein the floating structure is provided with a buoyant unit which is placed to float on a water surface to provide buoyancy.

22. The culture facility for photosynthetic microorganisms of claim 19, wherein the floating structure is provided with an operator supporting unit to allow an operator to conduct a management work.

23. A method for culturing photosynthetic microorganisms, comprising:
preparing the photobioreactor of any one of claims 1 to 18; and
introducing, into one or more of the photobioreactor, photosynthetic microorganisms and a culture medium, and then putting the photobioreactor to environmental water to perform photosynthesis.

24. The method of culturing photosynthetic microorganisms of claim 23, wherein the photosynthetic microorganisms are dispersed and cultured in a culture medium without a carrier.
